(19) Europäisches Patentamt
European Patent Office
Office européen des brevets

(11) Numéro de publication: **0 002 978**
**B1**

(12) **FASCICULE DE BREVET EUROPEEN**

(45) Date de publication du fascicule du brevet: **06.10.82**

(21) Numéro de dépôt: **78400236.2**

(22) Date de dépôt: **13.12.78**

(51) Int. Cl.³: **C 07 D 277/34,**
A 61 K 31/425

(54) **Dérivés de thiazolidinedione-2,4, leur préparation et leur application en thérapeutique.**

(30) Priorité: **29.12.77 FR 7739559**
**24.11.78 FR 7833244**

(43) Date de publication de la demande:
**11.07.79 Bulletin 79/14**

(45) Mention de la délivrance du brevet:
**06.10.82 Bulletin 82/40**

(84) Etats contractants désignés:
**BE CH DE FR GB IT LU NL SE**

(56) Documents cités:
**FR - A - 1 435 004**

**Chemical Abstracts Vol. 58 (1958) 7951.**

(73) Titulaire: **SYNTHELABO**
**58 rue de la Glacière**
**F-75013 Paris (FR)**

(72) Inventeur: **Bigg, Dennis Claude**
**24, rue Curie**
**F-34590 Marsillargues (FR)**

(74) Mandataire: **Thouret-Lemaitre, Elisabeth**
**Service Brevets - SYNTHELABO 58, rue de la Glacière**
**F-75621 Paris Cedex 13 (FR)**

**EP 0 002 978 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Dérivés de thiazolidinedione-2,4, leur préparation et leur application en thérapeutique

La présente invention a pour objet des dérivés de thiazolidinedione-2,4, sous forme de racémates ou d'énantiomères, leurs sels d'addition aux acides pharmaceutiquement acceptables, leur préparation et leur application en thérapeutique.

Les composés de l'invention répondent à la formule (I)

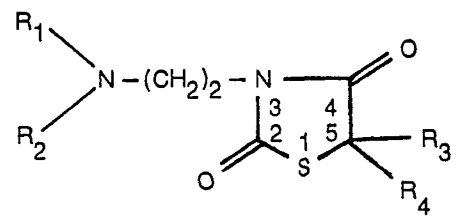

(I)

dans laquelle

$R_1$ est un atome d'hydrogène, un radical alkyle de 1 à 4 atomes de carbone ou le radical benzyle pouvant ou non porter un substituant choisi parmi les radicaux méthyle et méthoxy et les atomes d'halogène,

$R_2$ est un atome d'hydrogène,

$R_3$ est

soit un radical phényle pouvant ou non porter un ou plusieurs substituants choisis dans le groupe constitué par les atomes d'halogène, les radicaux alkyle et alcoxy de 1 à 4 atomes de carbone, les radicaux $CF_3$ et $CF_3S$,

soit un radical naphtyle,

et

$R_4$ est un atome d'hydrogène, un radical alkyle de 1 à 4 atomes de carbone, le radical phényle ou le radical benzyle.

Les sels d'addition des composés (I) aux acides pharmaceutiquement acceptables font partie de l'invention.

Les composés de l'invention comportent un carbone asymétrique et peuvent donc donner naissance à deux isomères optiquement actifs.

Ces isomères peuvent être préparés par toute méthode appropriée ou préparés par synthèse stéréospécifique et font partie de l'invention.

Des thiazolidinediones ont déjà été décrites dans C.A. Vol. 58, 7951 (1963). Elles répondent à la formule

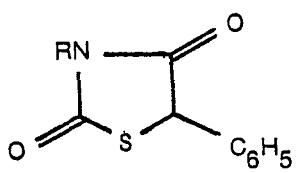

dans laquelle R est $(CH_3)_2N(CH_2)_3$, $(CH_3)_2N(CH_2)_2$, 2-morpholinoéthyle ou 2-pipéridinoéthyle. La Demanderesse a synthétisé le composé dans lequel R est $(CH_3)_2N(CH_2)_2$ et l'a comparé pharmacologiquement à ses propres composés de formule (I).

Il apparaît que ce composé n'est pas actif en tant qu'anti-convulsivant (DE50 $>100$) ni en tant qu'antidépresseur (DE50 $>10$) alors que les composés de formule (I) sont actifs.

Les composés préférés de l'invention sont ceux dans lesquels $R_1$ et $R_2$ sont des atomes d'hydrogène ou l'un des deux est le radical méthyle.

Parmi ceux-ci sont à considérer les composés pour lesquels $R_3$ est un radical phényle portant un ou plusieurs atomes d'halogène et/ou groupes $CF_3$ ou $CH_3O$.

Selon l'invention on prépare les composés selon le schéma réactionnel suivant:

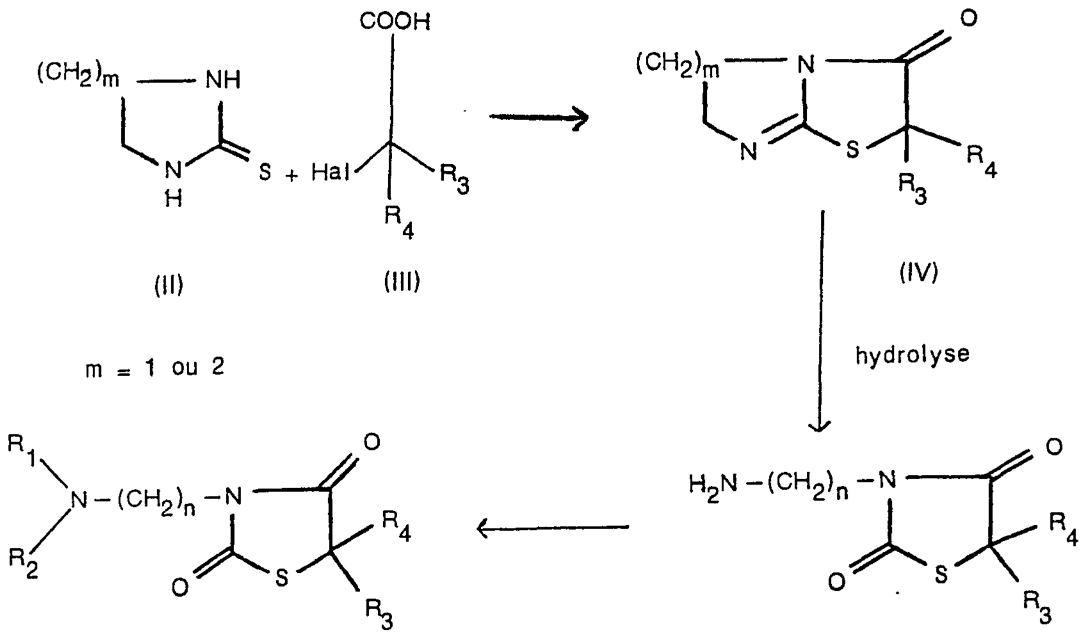


(II) (III) (IV)

m = 1 ou 2

(I) (I)

La condensation des composés (II) et (III) est effectuée de préférence dans de l'acide à une température de 60 à 80°C.

Lorsque $R_4$ est H, le procédé de l'invention consiste à faire réagir le composé (II) avec le composé (III)'

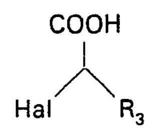 ($R_4 = H$)

puis à faire subir au composé obtenu (IV)'

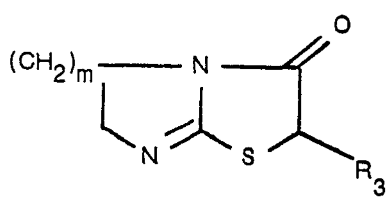

diverses transformations conduisant aux différents composés (I): voir schéma réactionnel ci-après

1. hydrolyse conduisant à (I) dans lequel $R_1 = R_2 = R_4 = H$ qui peut être alkylé en composé (I) dans lequel $R_1$ et $R_2$ peuvent être différents de H;

2.1 substitution en 2 du cycle imidazo [2,1-b] thiazole par $R_4$, puis hydrolyse conduisant à (I) dans lequel $R_1 = R_2 = H$

2.2 substitution en 2 du cycle imidazo [2,1-b] thiazole par $R_4$, puis substitution sur l'azote par $R_1$ et hydrolyse conduisant à (I) dans lequel $R_2 = H$

3. substitution sur l'azote par $R_1$ puis hydrolyse conduisant à (I) dans lequel $R_2 = R_4 = H$.

Les produits de départ (III)' sont connus.

Les intermédiaires (IV) sont nouveaux à l'exception de ceux dans lequels $R_4$ est H, n = 2 et $R_3$ est $C_6H_5$, 2-Cl—$C_6H_4$, 2,4-di-Cl—$C_6H_3$ et 3,4-di-Cl—$C_6H_3$.

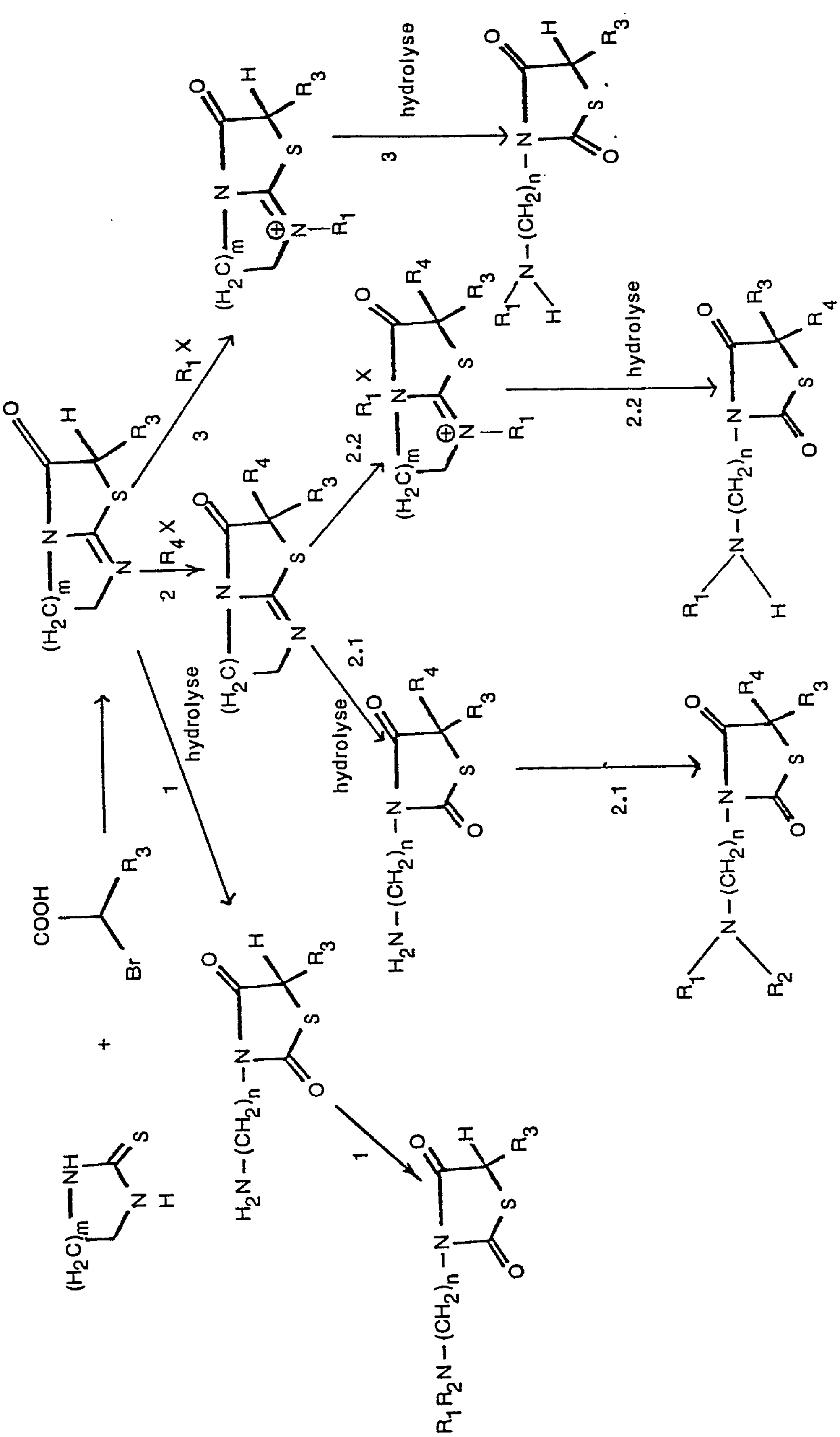

hydrolyse
3
$R_1X$
2
$R_4X$
2.2
$R_1X$
2.2
hydrolyse
2.1
hydrolyse
2.1
1
hydrolyse
1

Les exemples suivants illustrent l'invention.
Les analyses et spectres IR et RMN confirment la structure des composés.

Exemple 1
Bromhydrate d'(amino-2 éthyl)-3 méthyl-5 (fluoro-4 phényl)-5 thiazolidinedione-2,4.

$$[R_1 = R_2 = H \ R_3 = 4\text{-F—}C_6H_4 \ R_4 = CH_3]$$

1.1 bromhydrate de p-fluoro-phényl-2 méthyl-2 dihydro-5,6 imidazo [2,1-b] thiazole [2H]-one-3.

Dans un ballon de 1 l, on introduit 18,9 g (0.185 mole) d'éthylène-thiourée et 45,8 g (0,185 mole) d'acide $\alpha$-bromo $\alpha$-méthyl p-fluoro-phénylacétique dans 110 $cm^3$ d'acide acétique.

On chauffe à 65—75°C pendant 4 heures jusqu'à obtenir une solution limpide. On laisse refroidir et on concentre. On récupère une huile orange que l'on reprend dans de l'acétone. On obtient un solide que l'on filtre, lave à l'acétone, rince et sèche.

Après recristallisation dans de l'éthanol, on obtient un solide blanc.

F = 227—229°C

1.2 bromhydrate d'(amino-2 éthyl)-3 méthyl-5 (fluoro-4 phényl)-5 thiazolidinedione-2,4.

Dans un ballon de 250 ml on introduit 9.6 g du bromhydrate obtenu précédemment, 40 ml d'eau et 4 ml d'acide bromhydrique concentré.

On porte à reflux 6 heures. On obtient une solution limpide, jaune très clair. On chasse l'eau sous vide et récupère un solide blanc.

F = 227—228,5°C

Exemple 2
Bromhydrate d'(amino-2 éthyl)-3 phényl-5 thiazolidinedione-2,4.

$$[R_1 = R_2 = H \ R_3 = C_6H_5 \ R_4 = H]$$

1. bromhydrate de phényl-2 dihydro-5,6 imidazo ]2,1-b] thiazole ]2H] -one-3.

Dans un ballon de 250 $cm^3$, on introduit 10 g (0,098 mole) d'éthylène-thiourée et 21,05 g (0,098 mole) d'acide $\alpha$-bromo-phényl-acétique dans 50 $cm^3$ d'acide acétique.

On chauffe à 65—75°C pendant 4 heures. On laisse refroidir.

On filtre et sèche le précipité puis on le recristallise dans du méthanol.

Le solide blanc obtenu fond à 245—247°C en se décomposant.

2. bromhydrate d'(amino-2 éthyl)-3 phényl-5 thiazolidinedione-2,4.

Dans un ballon de 100 $cm^3$ on introduit 15 g du bromhydrate précédent, 20 $cm^3$ d'eau et 4 $cm^3$ d'acide bromhydrique concentré. On porte à reflux pendant 4 heures puis on chasse l'eau sous pression réduite. On obtient un solide blanc que l'on lave et recristallise dans de l'éthanol.

F = 220—222°C

Exemple 3
(acétylamino-2 éthyl)-3 phényl-5 thiazolidinedione-2,4.

$$[R_1 = CH_3CO \ R_2 = H \ R_3 = C_6H_5 \ R_4 = H]$$

L'(amino-2 éthyl)-3 phényl-5 thiazolidinedione-2,4 est obtenue à partir de son bromhydrate (exemple 2.2).

C'est un solide blanc qui fond à 139,5—140,5°C.

On introduit 8,9 g (0,038 mole) de cette base et 300 $cm^3$ de pyridine sèche dans un ballon. On agite 30 mn puis on ajoute lentement, sous argon, 3,14 g (0,04 mole) de chlorure d'acétyle.

Après 2 heures d'agitation, on chasse la pyridine. On récupère une huile jaune que l'on reprend avec du chloroforme. On obtient après lavage et concentration une huile jaune clair qui cristallise immédiatement dans de l'éther.

Le solide blanc obtenu, après recristallisation dans du toluène, fond à 136—137°C.

Exemple 4
Chlorhydrate d'(amino-2 éthyl)-3 méthyl-5 phényl-5 thiazolidinedione-2,4.

$$[R_1 = R_2 = H \ R_3 = CH_3 \ R_4 = C_6H_5]$$

4.1 phényl-2 méthyl-2 dihydro-5,6 imidazo [2,1-b] thiazole [2H] -one-3.

On ajoute une solution du composé obtenu sous 2.1 sous forme de base libre (32,7 g—0,15 mole) dans du DMF sec (400 ml) à de l'hydrure de sodium prélavé avec du pentane tout en refroidissant au bain de glace. On effectue l'addition en 30 mn sous atmosphère d'azote. On agite la suspension 1 heure à la température ambiante, on la refroidit au bain de glace et on ajoute de l'iodure de méthyle (21,3 g—0,15 mole) goutte à goutte en 5 mn.

On agite le mélange réactionnel pendant 3 heures et on verse la solution rouge clair dans de l'eau. On extrait avec de l'acétate d'éthyle jusqu'à ce que les extraits soient incolores, on lave avec de l'eau et sèche sur $MgSO_4$.

On filtre et évapore et obtient un solide jaune. Après recristallisation dans un mélange éther isopropylique/acétate d'éthyle le solide fond à 102—103,5°C.

4.2 chlorhydrate d'(amino-2 éthyl)-3 méthyl-5 phényl-5 thiazolidinedione-2,4.

On acidifie à pH-1—2 une suspension de la base libre obtenue précédemment (voir 4.1) (9,2 g—0,04 mole) dans 80 ml d'eau, à l'aide d'acide chlorhydrique 2N.

On chauffe la solution incolore à reflux pendant 6 heures.

On concentre le milieu réactionnel sous pression réduite.

On obtient une solide blanc que l'on recristallise dans de l'éthanol.

F = 223—224,5°C

Exemple 5

Chlorhydrate de (diméthylamino-2 éthyl)-3 méthyl-5 phényl-5 thiazolidinedione-2,4.

$[R_1 = R_2 = CH_3 \; R_3 = C_6H_5 \; R_4 = CH_3]$

On chauffe à 100°C pendant 12 heures un mélange du composé obtenu sous 4.2 (12,6 g—0,044 mole), d'acide formique (10.13 g—0,22 mole) et de formaldéhyde (8,6 ml d'une solution à 35%—0,01 mole).

On refroidit la solution incolore la dilue avec de l'eau et lave avec $CHCl_3$. On alcalinise avec $Na_2CO_3$ et extrait avec $CHCl_3$.

On lave les extraits, les sèche sur $MgSO_4$ et on évapore.

On obtient une huile que l'on reprend et traite avec $Et_2O/HCl$.

On filtre le précipité blanc et le recristallise dans un mélange EtOH/éther de pétrole puis dans de la méthyl-éthylcétone.

F = 178,5—179,5°C

Exemple 6

Bromhydrate de (benzylamino-2 éthyl)-3 méthyl-5 phényl-5 thiazolidinedione-2,4.

$[R_1 = C_6H_5CH_2 \; R_2 = H \; R_3 = C_6H_5 \; R_4 = CH_3]$

on ajoute du bromure de benzyle (5,13 g—0,013 mole) à une solution du composé obtenu sous 4.1 (6,96 g—0,03 mole) dans EtOAc (250 ml).

On chauffe à reflux le mélange pendant 5 jours et on filtre le précipité (A) de formule

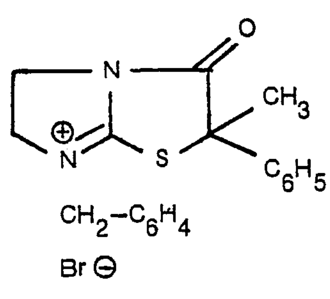

On peut obtenir d'avantage de composé A en chauffant le filtrat pendant 5 jours avec 0,03 mole de bromure de benzyle.

On reprend le composé A (9,3 g) dans un mélange éthanol/eau (25/25 ml) et on chauffe à reflux pendant 4 heures.

On refroidit la solution rouge clair, le traite sur du charbon et l'évapore.

Le solide blanc obtenu par trituration est recristallisé dans un mélange éthanol/éther de pétrole puis dans un mélange isopropanol/éthanol.

F = 169—170,5°C

Exemple 7

Chlorhydrate de (méthylamino-2 éthyl)-3 phényl-5 thiazolidinedione-2,4.

$$[R_1 = CH_3\ R_2 = H\ R_3 = C_6H_5\ R_4 = H]$$

On ajoute du sulfate de diméthyle (6,3 g—0,05 mole) à une suspension du composé obtenu sous 2.1 (10,9 g—0,05 mole) dans de l'acétate d'éthyle (250 ml) et on chauffe le mélange à reflux.

On refroidit le mélange réactionnel après *6* heures et on filtre.

Le méthylsulfate fond à 161—4°C.

On dissout le produit cristallin blanc dans de l'eau (50 ml) et on chauffe à reflux pendant 6 heures. On amène la solution jaune clair à pH 2—3 et on l'extrait avec du chloroforme. On évapore les extraits séchés sur $MgSO_4$ et on reprend l'huile dans de l'acétone. On la transforme en chlorhydrate.

Après recristallisation dans de l'isopropanol puis dans un mélange éthanol/éther de pétrole on obtient un solide blanc.

F = 226,5—228,5°C

Exemple 8

Bromhydrate d'(amino-2 éthyl)-3 (naphtyl-1)-5 thiazolidinedione-2,4.

1.1 bromhydrate de (naphtyl-1)-2 dihydro-5,6 imidazo [2,1-b] thiazole [2H] one-3.

Dans un ballon de 1 l, on introduit 42,4 g (0,415 mole) d'éthylène thiourée et 110 g (0,415 mole) de l'acide $\alpha$-bromo-(naphtyl-1)-acétique.

On chauffe à 65—75°C pendant 4 heures. On filtre le solide obtenu que l'on recristallise dans un mélange méthanol/eau.

F = 304—306°C

1.2 bromhydrate d'(amino-2 éthyl)-3 (naphtyl-1)-5 thiazolidinedione-2,4.

Dans un ballon de 250 ml, on introduit 12 g du bromhydrate obtenu sous 1,1, 80 ml d'eau et 8 ml de HBr. On porte à reflux pendant 6 heures. On concentre sous vide la solution obtenue. On recristallise le solide obtenu dans de l'alcool isopropylique.

F = 142—144°C

Exemple 9

Bromhydrate d'(amino-2 éthyl)-3 (dichloro-3,4 phényl)-5 thiazolidinedione-2,4.

$$[R_1 = R_2 = H\ R_3 = 3,4\text{-}Cl_2\text{—}C_6H_3\ R_4 = H]$$

1.1 bromhydrate de (dichloro-3,4 phényl)-2 dihydro-5,6 imidazo [2,1-b] thiazole 2H-one-3.

Dans un ballon de 250 $cm^3$, on introduit 2,0 g (0,02 mole) d'éthylène-thiourée et 6,5 g (0,023 mole) d'acide $\alpha$-bromo-3,4-dichlorophénylacétique dans 15 $cm^3$ d'acide acétique.

On chauffe à 65—75°C pendant 4 heures. Après 1/2 heure, la solution devient limpide, puis prend en masse. On récupère un solide que l'on rince à l'acétone.

F = 265—267°C (déc.)

1.2 bromhydrate d'(amino-2 éthyl)-3 (dichloro-3,4 phényl)-5 thiazolidinedione-2,4.

Dans un ballon de 250 $cm^3$, on introduit 6,2 g (0,0168) de bromhydrate, 60 $cm^3$ d'eau et 3 $cm^3$ d'acide bromhydrique concentré. On porte à la température du reflux pendant 6 heures jusqu'à obtention d'une solution limpide incolore.

On concentre sous vide à 90°C. On obtient un solide blanc.

On le rince à chaud avec de l'acétate d'éthyle et on filtre. le solide blanc obtenu, est recristallisé dans un mélange acétone/éthanol (2/1), puis une autre fois dans de l'éthanol.

F = 226—228°C

Dans le tableau suivant (I) sont rassemblés les composés de l'invention préparés à titre d'exemples.

TABLEAU I

| Composé n° | $R_1$ | $R_2$ | $R_3$ | $R_4$ | n | sel | F(°C) |
|---|---|---|---|---|---|---|---|
| 1 | H | H | $C_6H_5$ | H | 2 | HBr | 220—222 |
| 2 | $C_6H_5CO$ | H | $C_6H_5$ | H | 2 | — | 156—7 |
| 3 | $CH_3CO$ | H | $C_6H_5$ | H | 2 | — | 136—7 |
| 4 | H | H | $C_6H_5$ | H | 3 | HBr | 194—195,5 |
| 5 | H | H | $C_6H_5$ | $C_6H_5CH_2$ | 2 | $(CO_2H)_2 1H_2O$ | 149—150 |
| 6 | H | H | $C_6H_5$ | $CH_3$ | 2 | HCl | 223—224,5 |
| 7 | H | H | $C_6H_5$ | $C_6H_5$ | 2 | HCl | 205—206,5 |
| 8 | H | H | 4-Cl$C_6H_4$ | H | 2 | HBr | 184—185—5 |
| 9 | H | H | 4-MeO$C_6H_4$ | H | 2 | HBr | 176—177,5 |
| 10 | H | H | 4-Cl$C_6H_4$ | H | 3 | HBr | 183—5 |
| 11 | H | H | 4-MeO$C_6H_4$ | H | 3 | HBr | 217—218,5 |
| 12 | $C_6H_5CH_2$ | H | $C_6H_5$ | H | 2 | $(CO_2H)_2$ | 227—228 |
| 13 | $CH_3$ | H | $C_6H_5$ | H | 2 | HCl | 226,5—228,5 |
| 14 | $CH_3$ | H | $C_6H_5$ | $CH_3$ | 2 | HCl | 162—163,5 |

TABLEAU I (suite)

| Composé n° | $R_1$ | $R_2$ | $R_3$ | $R_4$ | n | sel | F (°C) |
|---|---|---|---|---|---|---|---|
| 15 | $C_6H_5CH_2$ | H | $C_6H_5$ | $CH_3$ | 2 | HBr | 169—170,5 |
| 16 | H | H | 4-$FC_6H_4$ | H | 2 | HBr | 200—201,5 |
| 17 | H | H | 4-$BrC_6H_5$ | H | 2 | HBr | 213—214,5 |
| 18 | $CH_3$ | $CH_3$ | $C_6H_5$ | $CH_3$ | 2 | HCl | 178—179,5 |
| 19 | H | H | 4-$ClC_6H_4$ | $CH_3$ | 2 | HCl | 204—5 |
| 20 | H | H | 4-$ClC_6H_4$ | $C_2H_5$ | 2 | HCl | 248,5—250 |
| 21 | H | H | 4-$ClC_6H_4$ | *n*-$C_3H_7$ | 2 | HCl | 250—2 |
| 22 | H | H | 4-$ClC_6H_4$ | $H_2C{=}CHCH_2$ | 2 | HCl | 228—9 |
| 23 | H | H | 4-$FC_6H_4$ | $CH_3$ | 2 | HBr | 227—228,5 |
| 24 | H | H | 1 naphtyl | H | 2 | HBr | 142—4 |
| 25 | H | H | 2-$ClC_6H_4$ | H | 2 | HBr | 226—227,5 |
| 26 | H | H | 4-$CH_3C_6H_4$ | H | 2 | HCl1/2$H_2O$ | 190—192,5 |
| 27 | H | H | 3-$CF_3C_6H_4$ | H | 2 | HCl | 185—186,5 |
| 28 | H | H | 3-$MeOC_6H_4$ | H | 2 | HCl | 218,5—220 |

TABLEAU I (suite)

| Composé n° | $R_1$ | $R_2$ | $R_3$ | $R_4$ | n | sel | F (°C) |
|---|---|---|---|---|---|---|---|
| 29 | H | H | $3\text{-}NO_2C_6H_4$ | H | 2 | HCl | 216—217,5 |
| 30 | H | H | $C_6H_5$ | $C_2H_5$ | 2 | HCl | 202—203,5 |
| 31 | H | H | $C_6H_5$ | iso $C_3H_7$ | 2 | HCl1/2$H_2O$ | 144—145—5 |
| 32 | H | H | $C_6H_5$ | n-$C_3H_7$ | 2 | HCl | 217—218,5 |
| 33 | $CH_3$ | $CH_3$ | 1-naphtyl | H | 2 | HCl1/4$H_2O$ | 213—215 |
| 34 | H | H | 1-naphtyl | $CH_3$ | 2 | HCl1/2$H_2O$ | 167,5—169 |
| 35 | $C_6H_5CH_2$ | H | 1-naphtyl | H | 2 | HBr1/4$H_2O$ | 190—1 |
| 36 | H | H | 3-Cl-$C_6H_4$ | H | 2 | HBr | 189—190,5 |
| 37 | $C_6H_5CH_2$ | H | $3\text{-}CF_3C_6H_4$ | H | 2 | HBr | 136—8 |
| 38 | H | H | 2,4-$Cl_2$—$C_6H_3$ | H | 2 | HBr | 216,5—218 |
| 39 | H | H | 3,4-$Cl_2$—$C_6H_3$ | H | 2 | HBr | 226—8 |
| 40 | H | H | 3-$CF_3S$—$C_6H_4$ | H | 2 | HBr | 162—3,5 |
| 41 | 2-$CH_3$—$C_6H_4CH_2$ | H | 3-$CH_3O$—$C_6H_4$ | H | 2 | HBr | 145,5—147 |
| 42 | H | H | 4-F,3-$CF_3$—$C_6H_3$ | H | 2 | HBr | 122—4 |
| 43 | $CH_3$ | H | 3,4-$Cl_2$—$C_6H_3$ | H | 2 | HCl | 249—251 |
| 44 | $CH_3$ | H | 3-$CF_3$—$C_6H_4$ | H | 2 | HCl | 181—3 |
| 45 | H | H | 2-Br-4,5-di $CH_3O$—$C_6H_2$ | H | 2 | HCl | 216—8 |
| 46 | $C_2H_5$ | H | 3-$CF_3$—$C_6H_4$ | H | 2 | HCl | 147,5—9 |

Les composés de l'invention ont été soumis à des essais pharmacologiques, qui ont révélé une activité anticonvulsivante et pour certains d'entre eux une activité antidépressive.

La toxicité aigüe a été déterminée chez la souris par voie intrapéritonéale. Elle varie de 200 à 1000 mg/kg.

L'activité anticonvulsivante a été déterminée par le test de l'antagonisme vis à vis des convulsions induites par la bicuculline chez la souris (Perez de la Mora, M. (1973) and Tapia R Biochem. Pharmacol. *22*, 2635—2639).

Les produits à étudier sont injectés par voie intrapéritonéale, 30 mn avant la bicuculline (0,9 mg/kg *i.v.*).

Le critère retenu pour ce test étant la léthalité, les pourcentages de mortalité sont notés pour chaque lot, 2 heures après administration de la bicuculline (lot témoins: 100% mortalité).

Pour chaque produit, la dose active 50% (DA 50 ou dose protégeant 50% d'animaux des effets léthaux de la bicuculline) est déterminée graphiquement.

La DA 50 des composés de l'invention varie entre 20 et 60 mg/kg par voie i.p.

L'activité antidépressive a été déterminée selon le test de l'antagonisme de la ptose réserpinique (Gouret C. et al., J. Pharmacol. (Paris) *8*, 333—350 (1977).

Les souris (mâles, CD1 Charles River, France, 18—22 g), reçoivent stimultanément les produits à étudier ou le solvant (*voie i.p.*), et la réserpine (4 mg/kg, *voir s.c*).

Soixante minutes plus tard, le degré de ptose palpébrale est estimé au moyen d'une échelle de cotation (0 à 4) pour chaque souris.

A chaque dose, la moyenne de cotation et le % de variation par rapport au lot contrôle, sont calculés.

Pour chaque produit, la DA 50, ou dose qui diminue de 50% le score moyen de ptose par rapport aux contrôles, est déterminée graphiquement.

La DA des composés de l'invention va de 1,5 à 10 mg/kg.

Les résultats des tests montrent que les composés de l'invention sont des anticonvulsivants utiles pour le traitement des différents types d'épilepsies, et des antidépresseurs utiles pour le traitement de la dépression.

Les composés de l'invention peuvent être présentés sous toute forme appropriée pour l'administration par voie orale, parentérale, endorectale, par exemple sous forme de comprimés, de dragées, de gélules, de solutions buvables ou injectables, etc ... avec tout excipient approprié.

La posologie quotidienne peut aller de 200 à 1500 mg/kg pour les anticonvulsivants et de 5 à 200 mg pour les antidépresseurs.

**Revendications**

1. Dérivés de thiazolidinedione-2,4, sous la forme de racémates ou d'isomères optiquement actifs, répondant à la formule (I)

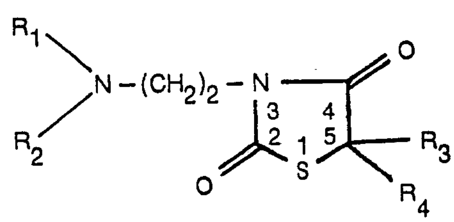

dans laquelle

$R_1$ est un atome d'hydrogène, un radical alkyle de 1 à 4 atomes de carbone ou le radical benzyle pouvant ou non porter un substituant choisi parmi les radicaux méthyle et méthoxy et les atomes d'halogène,

$R_2$ est un atome d'hydrogène,

$R_3$ est

soit un radical phényle pouvant ou non porter un ou plusieurs substituants choisis dans le groupe constitué par les atomes d'halogène, les radicaux alkyle et alcoxy de 1 à 4 atomes de carbone, les radicaux $CF_3$ et $CF_3S$,

soit un radical naphtyle,

et

$R_4$ est un atome d'hydrogène, un radical alkyle de 1 à 4 atomes de carbone, le radical phényle ou le radical benzyle, ainsi que leurs sels d'addition aux acides pharmaceutiquement acceptables.

2. Dérivés selon la revendication 1, dans lesquels $R_1$ et $R_2$ sont des atomes d'hydrogène..

3. Dérivés selon la revendication 1, dans lesquels $R_3$ est un radical phényle portant un ou plusieurs atomes d'halogène et/ou groupes $CF_3$ ou $CH_3O$ et $R_1$, $R_2$ et $R_4$ ont les significations données dans la revendication 1.

4. Dérivés selon la revendication 1, dans lesquels

$R_1$ est H, $CH_3$, $C_2H_5$, $C_6H_5CH_2$,

$R_2$ est H,

$R_3$ est $C_6H_5$, 4-Cl—$C_6H_4$, 4-F—$C_6H_4$, 4-Br—$C_6H_4$, 4-$CH_3O$—$C_6H_4$, 2-Cl—$C_6H_4$, naphtyl-1, 3-$CF_3$—$C_6H_4$, 3-$CF_3S$—$C_6H_4$, 3-$CH_3O$—$C_6H_4$, 3-Cl—$C_6H_4$, 2,4-$Cl_2$—$C_6H_3$, 3,4-$Cl_2$—$C_6H_3$, 3-$CF_3$, 4-F—$C_6H_3$,

et

$R_4$ est H.

5. L'(amino-2 éthyl)-3 (dichloro-3,4-phényl)-5 thiazolidinedione-2,4 et ses sels pharmaceutiquement acceptables.

6. Le (méthylamino-2 éthyl)-3 (dichloro-3,4 phényl)-5 thiazolidinedione-2,4 et ses sels pharmaceutiquement acceptables.

7. L'(amino-2 éthyl)-3 (trifluorométhyl-3 phényl)-5 thiazolidinedione-2,4 et ses sels pharmaceutiquement acceptables.

8. L'(amino-2 éthyl)-3(naphtyl-1)-5 thiazolidinedione-2,4 et ses sels pharmaceutiquement acceptables.

9. Le (méthylamino-2 éthyl)-3 (trifluorométhyl-3 phényl)-5 thiazolidinedione-2,4 et ses sels pharmaceutiquement acceptables.

10. Procédé d'analogie de préparation des composés selon la revendication 1, procédé caractérisé en ce qu'on fait réagir un composé de formule (II)

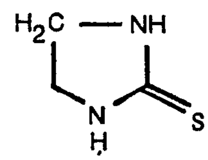

avec un acide bromé de formule (III)

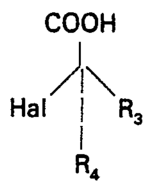

puis on hydrolyse le composé obtenu de formule (IV)

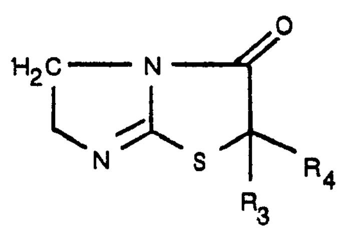

et si on le désire, on alkyle le composé (I) obtenu dans lequel $R_1$ et $R_2$ sont H; les symboles ayant les significations données dans la revendication 1.

11. Médicament caractérisé en ce qu'il contient un composé tel que spécifié dans l'une quelconque des revendications 1 à 9.

**Claims**

1. Thiazolidinedione-2,4 derivatives, in form of racemates or optically active isomers, corresponding to formula (I)

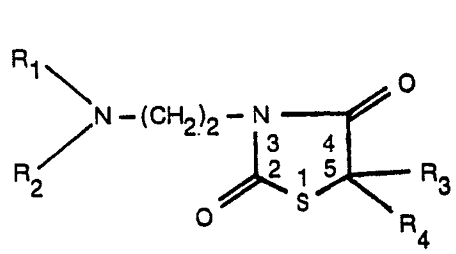

wherein

$R_1$ is a hydrogen atom, an alkyl radical having 1 to 4 carbon atoms or a benzyl radical having no substituent or having a substituent selected from methyl and methoxy groups and halogen atoms,

$R_2$ is a hydrogen atom,

$R_3$ is either a phenyl radical having no substituent or having one or more substituents selected from halogen atoms, alkyl and alcoxy radicals having 1 to 4 carbon atoms, and $CF_3$ and $CF_3S$ radicals, or a naphtyl radical,

and

$R_4$ is a hydrogen atom, an alkyl radical having 1 to 4 carbon atoms, a phenyl radical or a benzyl radical,

and also their addition salts with pharmaceutically acceptable acids.

2. Derivatives according to claim 1, wherein $R_1$ and $R_2$ are hydrogen atoms.

3. Derivatives according to claim 1, wherein $R_3$ is a phenyl radical carrying one or more halogen atoms and/or $CF_3$ or $CH_3O$ groups and $R_1$, $R_2$ and $R_4$ are defined as in claim 1.

4. Derivatives according to claim 1, wherein

$R_1$ is H, $CH_3$, $C_2H_5$, $C_6H_5CH_2$,

$R_2$ is H,

$R_3$ is $C_6H_5$, 4-Cl—$C_6H_4$, 4-F—$C_6H_4$, 4-Br—$C_6H_4$, 4-$CH_3O$—$C_6H_4$, 2-Cl—$C_6H_4$, naphtyl-1, 3-$CF_3$—$C_6H_4$, 3-$CF_3S$—$C_6H_4$, 3-$CH_3O$—$C_6H_4$, 3-Cl—$C_6H_4$, 2,4-$Cl_2$—$C_6H_3$, 3,4-$Cl_2$—$C_6H_3$, 3-$CF_3$, 4-F—$C_6H_3$,

and

$R_4$ is H.

5. 3-(2-Amino-ethyl)-5-(3,4-dichloro-phenyl)-thiazolidinedione-2,4, and its pharmaceutically acceptable salts.

6. 3-2(Methylamino-ethyl)-5-(3,4-dichloro-phenyl)-thiazolidinedione-2,4, and its pharmaceutically acceptable salts.

7. 3-(2-Amino-ethyl)-5-(3-trifluoromethyl-phenyl)-thiazolidinedione-2,4, and its pharmaceutically acceptable salts.

8. 3-(2-Amino-ethyl)-5-(1-naphtyl)-thiazolidinedione-2,4, and its pharmaceutically acceptable salts.

9. 3-(2-Methylamino-ethyl)-5-(3-trifluoromethyl-phenyl)-thiazolidinedione-2,4, and its pharmaceutically acceptable salts.

10. A process of analogy for preparing the compounds according to claim 1, characterized in that a compound of formula (II)

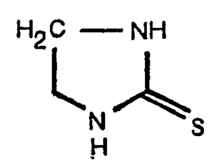

is reacted with a brominated acid of formula (III)

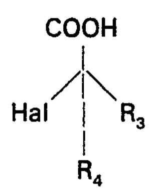

then the obtained compound of formula (IV)

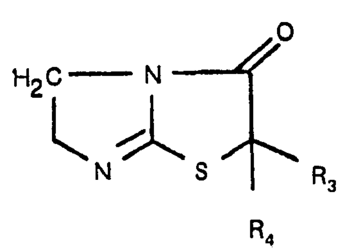

is hydrolyzed and, if desired, the obtained compound of formula (I), wherein $R_1$ and $R_2$ are H, is alkylated, the symbols being defined as in claim 1.

11. A drug characterized in that it contains a compound as specified in any one of claims 1 to 9.

**Patentansprüche**

1. Thiazolidindion-2,4 Derivate, in Form derer Racemate oder optisch aktiven Isomere, der allgemeinen Formel

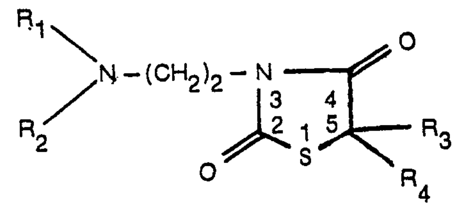

worin

$R_1$ ein Wasserstoffatom, einen Alkylrest mit 1 bis 4 Kohlenstoffatomen oder einen gegebenenfalls durch einen Methylrest, einen Methoxyrest oder ein Halogenatom substituierten Benzylrest,

$R_2$ ein Wasserstoffatom,

$R_3$ entweder einen gegebenenfalls durch ein oder mehrere Halogenatome, Alkyl- und Alkoxyreste mit 1 bis 4 Kohlenstoffatomen, $CF_3$-Reste und $CF_3S$-Reste substituierten Phenylrest, oder einen Naphtylrest,

und

$R_4$ ein Wasserstoffatom, einen Alkylrest mit 1 bis 4 Kohlenstoffatomen, einen Phenylrest oder einen Benzylrest bedeutet,

sowie deren Additionssalze mit pharmazeutisch annehmbaren Säuren.

2. Derivate gemäss Anspruch 1, worin $R_1$ und $R_2$ Wasserstoffatome bedeuten.

3. Derivate gemäss Anspruch 1, worin $R_3$ einen ein-oder mehrfach durch Halogenatome und/oder $CF_3$ oder $CH_3O$-gruppen substituierten Phenylrest bedeutet, und $R_1$, $R_2$ und $R_4$ die in Anspruch 1 angegebenen Bedeutungen haben.

4. Derivate gemäss Anspruch 1, worin

$R_1$ für H, $CH_3$, $C_2H_5$ oder $C_6H_5CH_2$,

$R_2$ für H,

$R_3$ für $C_6H_5$, 4-Cl—$C_6H_4$, 4-F—$C_6H_4$, 4-Br—$C_6H_4$, 4-$CH_3O$—$C_6H_4$, 2-Cl—$C_6H_4$, Naphtyl-1, 3-$CF_3$—$C_6H_4$, 3-$CF_3S$—$C_6H_4$, 3-$CH_3O$—$C_6H_4$, 3-Cl—$C_6H_4$, 2,4-$Cl_2$—$C_6H_3$, 3,4-$Cl_2$—$C_6H_3$ oder 3-$CF_3$, 4-F—$C_6H_3$,

und,

$R_4$ für H steht.

5. 3-(2-Amino-äthyl)-5-(3,4-dichloro-phenyl)-thiazolidindion-2,4, und dessen pharmazeutisch annehmbaren Salze.

6. 3-(2-Methylamino-äthyl)-5-(3,4-dichloro-phenyl)-thiazolidindion-2,4 und dessen pharmazeutisch annehmbaren Salze.

7. 3-(2-Amino-äthyl)-5-(3-trifluoromethyl-phenyl)-thiazolidindion-2,4 und dessen pharmazeutisch annehmbaren Salze.

8. 3-(2-Amino-äthyl-5-(1-naphtyl)-thiazolidindion-2,4 und dessen pharmazeutisch annehmbaren Salze.

9. 3-(2-Methylamino-äthyl)-5-(3-trifluoromethyl-phenyl)-thiazolidindion-2,4 und dessen pharmazeutisch annehmbaren Salze.

10. Analogieverfahren zur Herstellung der Verbindungen gemäss Anspruch 1, dadurch gekennzeichnet, dass man eine Verbindung der Formel (II)

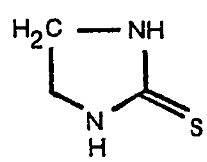

mit einer bromierten Säure der Formel (III)

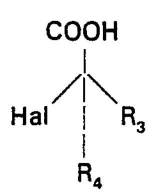

reagieren lässt,

danach die erhaltene Verbindung der Formel (IV)

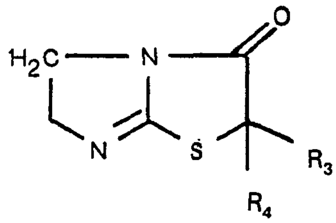

hydrolysiert,

und gewünschtenfalls die erhaltene Verbindung (I), worin $R_1$ und $R_2$ für H stehen, alkyliert, wobei die Symbole die in Anspruch 1 angegebenen Bedeutungen haben.

11. Arzneimittel, dadurch gekennzeichnet, dass es eine Verbindung gemäss einem der Ansprüche 1 bis 9 enthält.